# EUROPÄISCHE PATENTANMELDUNG

(11) **EP 2 284 518 A1**
(43) Veröffentlichungstag der Anmeldung: **16.02.2011**
(21) Anmeldenummer: 10166824.2
(22) Anmeldetag: 22.06.2010
(51) Int. Cl.: G01N 21/27, G01N 21/31

(54) **Verfahren zur Bestimmung eines Parameters, insbesondere des chemischen Sauerstoffbedarfs (CSB), des organischen Gesamtkohlenstoffgehalts (TOC) oder der Konzentration eines oder mehrerer Inhaltsstoffe, einer Flüssigkeitsprobe**

(30) Priorität: 31.07.2009 DE 102009028176; 06.08.2009 DE 102009028295
(71) Anmelder: Endress+Hauser Conducta Gesellschaft für Mess- und Regeltechnik mbH+Co. KG, 70839 Gerlingen (DE)
(72) Erfinder: Gahr, Achim, 63733, Goldbach (DE); Wenzel, Thomas, 64832, Gross-Umstadt (DE)
(74) Vertreter: Andres, Angelika Maria

(57) **Zusammenfassung**

Ein Verfahren zur Bestimmung eines Parameters, insbesondere des chemischen Sauerstoffbedarfs (CSB) oder des organischen Gesamtkohlenstoffgehalts (TOC), einer Flüssigkeitsprobe mittels einer spektrometrischen Vorrichtung, umfasst die Schritte:
Ermitteln eines Absorptionsspektrums der Flüssigkeitsprobe mindestens in einem vorgegebenen Wellenlängenbereich;
Ermitteln eines Messwerts des Parameters der Flüssigkeitsprobe aus dem Absorptionsspektrum im vorgegebenen Wellenlängenbereich;
Vergleichen des ermittelten Absorptionsspektrums im vorgegebenen Wellenlängenbereich mit mindestens einem Referenz-Absorptionsspektrum mindestens einer Referenzprobe bekannter Zusammensetzung;
Anhand des Vergleichs Bestimmen mindestens eines Ähnlichkeitsparameters, der die Ähnlichkeit zwischen dem ermittelten Absorptionsspektrum und dem Referenz-Absorptionsspektrum repräsentiert;
Klassifizieren des ermittelten Messwerts anhand des mindestens einen Ähnlichkeitsparameters.

## Beschreibung

Die Erfindung betrifft ein Verfahren zur Bestimmung eines Parameters, insbesondere des chemischen Sauerstoffbedarfs (CSB), des organischen Gesamtkohlenstoffgehalts (TOC) oder der Konzentration eines oder mehrerer Inhaltsstoffe, einer Flüssigkeitsprobe mittels einer spektroskopischen Messung, insbesondere mit Hilfe eines Spektrometers bzw. einer spektrometrischen Vorrichtung, worunter z.B. eine spektrometrische Messsonde oder ein spektrometrischer Sensor zu verstehen sind.

Der chemische Sauerstoffbedarf, kurz CSB, (auch englischer Fachbegriff: chemical oxygen demand, COD) ist die als Sauerstoffäquivalent ausgedrückte Menge an einer chemischen Verbindung, üblicherweise einem starken Oxidationsmittel, wie zum Beispiel Kaliumpermanganat oder Kaliumdichromat, die von den in einem bestimmten Volumen einer Flüssigkeitsprobe enthaltenen oxidierbaren Inhaltsstoffen unter den Reaktionsbedingungen einer vorgeschriebenen Methode verbraucht wird. Der CSB-Wert ist ein wichtiger Parameter zur Klassifizierung des Verschmutzungsgrads bei Fließwässern und in Abwasser- und Kläranlagen, insbesondere mit organischen Verunreinigungen. Ein verwandter Parameter ist der organische Gesamtkohlenstoffgehalt (auch englischer Fachbegriff: total organic carbon, TOC). Dieser Parameter repräsentiert den gesamten, in organischen Verbindungen enthaltenen Kohlenstoff in einer Flüssigkeitsprobe.

Bei den meisten Verfahren zur Bestimmung des chemischen Sauerstoffbedarfs wird eine Probe mit einem bekannten Überschuss eines Oxidationsmittels behandelt und anschließend der Verbrauch an Oxidationsmittel, beispielsweise durch Rücktitration des nicht verbrauchten Restes, ermittelt. Die Menge an verbrauchtem Oxidationsmittel wird in die äquivalente Sauerstoffmenge umgerechnet, die den CSB-Wert darstellt. Der organische Gesamtkohlenstoffgehalt wird häufig nach einem Verfahren bestimmt, bei dem eine Flüssigkeitsprobe nach Abtrennen des anorganisch gebundenen Kohlenstoffes in Anwesenheit von Sauerstoff verbrannt wird, so dass der gesamte organisch gebundene Kohlenstoff zu Kohlendioxid (CO₂) umgesetzt ist. Die so entstandene Menge an Kohlendioxid wird mittels eines Infrarotdetektors bestimmt.

Diese Verfahren liefern sehr genaue Ergebnisse. Nachteilig an diesen Verfahren ist jedoch, dass ihre Anwendung beispielsweise in industriellen Prozessen oder in Kläranlagen mit verhältnismäßig hohem apparativem Aufwand und Betreuungsaufwand verbunden ist. Nachteilig ist auch, insbesondere bei der CSB-Bestimmung, dass die zum Einsatz kommenden analytischen Methoden auf Standardverfahren beruhen, und daher meist nicht ohne Chemikalien auskommen. Gerade die weiter oben genannten Oxidationsmittel müssen in der Regel aufwändig entsorgt oder aufbereitet werden.

Eine dagegen verhältnismäßig einfache, in-situ oder online-Bestimmung der Parameter CSB oder TOC kann mit Hilfe von spektrometrischen Verfahren durchgeführt werden. Diese Verfahren können auch dazu genutzt werden, die Trübung oder die Konzentration eines oder mehrerer vorgegebener Inhaltsstoffe einer Flüssigkeitsprobe, wie z.B. Nitrat, zu bestimmen. In dem europäischen Patent EP 1 472 521 B1 ist ein dazu geeignetes Verfahren offenbart, bei dem ein längsbeweglicher Kolben oder Kolbenschieber die zu untersuchende Flüssigkeitsprobe in einen als Messraum dienenden Glaszylinder saugt und nach erfolgter Messung wieder aus diesem entfernt. Eine Lichtquelle und ein Lichtempfänger sind einander gegenüberliegend so angeordnet, dass die optische Achse des Strahlengangs zwischen Lichtquelle und Lichtempfänger, der Messpfad, im wesentlichen senkrecht zur Zylinderachse des Glaszylinders verläuft. Der zwischen Lichtquelle und Lichtempfänger verlaufende Messpfad wird auf diese Weise durch die zu untersuchende Flüssigkeitsprobe geführt. Bei der Lichtquelle kann es sich beispielsweise um eine UV-Lichtquelle handeln, z.B. um eine Blitzlampe. Der Lichtempfänger umfasst ein Spektrometer mit einem dispergierenden Element, welches das empfangene Licht wellenlängenabhängig auf eine Photodiodenzeile oder ein Photodiodenfeld abbildet. Auf diese Weise kann ein UV-Absorptionsspektrum der Flüssigkeitsprobe aufgenommen werden.

Mit diesem oder ähnlichen anderen Verfahren können, neben anderen Messgrößen wie Trübung oder Nitratkonzentration der Flüssigkeitsprobe, auch die CSB- oder TOC-Werte der Flüssigkeitsprobe ermittelt werden. Hierzu kann das Absorptionsspektrum oder auch allein der CSB- bzw. TOC-Beitrag zum Absorptionsspektrum über einen vorgegebenen UV-Wellenlängenbereich, beispielsweise zwischen 240 und 400 nm, integriert und aus dem ermittelten Integral-Wert anhand einer durch Kalibriermessungen an Referenzproben gewonnenen Zuordnungsvorschrift ein CSB-oder TOC-Wert der Flüssigkeitsprobe abgeleitet werden.

Dieses Verfahren funktioniert zuverlässig, so lange die grundsätzliche Zusammensetzung der Flüssigkeitsproben im wesentlichen gleich der Zusammensetzung der Referenzproben ist, mit denen die Kalibriermessungen durchgeführt wurden. Ändert sich jedoch die Zusammensetzung der Flüssigkeitsproben während der Einsatzdauer der Sonde signifikant, z.B. wenn in ein überwachtes Gewässer eine vorher nicht vorhandene Substanz in hoher Konzentration eingeleitet wird, können die mit den hinterlegten Kalibrierparametern ermittelten CSB- oder TOC-Werte erheblich von den tatsächlichen Werten der Flüssigkeitsproben abweichen. Dies wird jedoch bei dem aus dem Stand der Technik bekannten Verfahren nicht bemerkt, da anhand von Absorptionsmessungen allein nicht zu unterscheiden ist, ob eine festgestellte Änderung der Absorption auf eine reine Konzentrationsänderung bei im wesentlichen gleich bleibender Zusammensetzung oder auf eine Änderung der Zusammensetzung, z.B. durch einen neu hinzukommenden Bestandteil, zurückzuführen ist. Dies ergibt sich daraus, dass ein Absorptionsspektrum ein Summensignal aller in der Probe lichtabsorbierenden Substanzen ist, wobei diese Substanzen in unterschiedlicher Konzentration vorliegen können, und wobei die auf die Stoffmengeneinheit bezogene Stärke der Absorption von der chemischen Struktur der lichtabsorbierenden Substanzen abhängig ist, und daher die stoffmengenbezogene Stärke der Absorption unterschiedlich sein kann.

Es ist deshalb Aufgabe der Erfindung, ein Verfahren zur Bestimmung eines Parameters, insbesondere des chemischen Sauerstoffbedarfs, des organischen Gesamtkohlenstoffgehalts oder der Konzentration eines vorgegebenen Inhaltsstoffes einer Flüssigkeitsprobe anzugeben, das diese Nachteile überwindet. Insbesondere soll ein Verfahren angegeben werden, das eine Einschätzung der Verlässlichkeit des ermittelten Messwerts des Parameters erlaubt.

Diese Aufgabe wird gelöst durch ein Verfahren zur Bestimmung eines Parameters, insbesondere des chemischen Sauerstoffbedarfs (CSB), des organischen Gesamtkohlenstoffgehalts (TOC) oder der Konzentration eines oder mehrerer vorgegebener Inhaltsstoffe einer Flüssigkeitsprobe, einer Flüssigkeitsprobe mittels einer spektrometrischen Vorrichtung, umfassend die Schritte:
Ermitteln eines Absorptionsspektrums der Flüssigkeitsprobe mindestens in einem vorgegebenen Wellenlängenbereich;
Ermitteln eines Messwerts des Parameters der Flüssigkeitsprobe aus dem Absorptionsspektrum im vorgegebenen Wellenlängenbereich;
Vergleichen des ermittelten Absorptionsspektrums im vorgegebenen Wellenlängenbereich mit mindestens einem Referenz-Absorptionsspektrum mindestens einer Referenzprobe bekannter Zusammensetzung;
Anhand des Vergleichs Bestimmen mindestens eines Ähnlichkeitsparameters, der die Ähnlichkeit zwischen dem ermittelten Absorptionsspektrum und dem Referenz-Absorptionsspektrum repräsentiert;
Klassifizieren des ermittelten Messwerts anhand des mindestens einen Ähnlichkeitsparameters.

Die durch den mindestens einen Ähnlichkeitsparameter repräsentierte Ähnlichkeit zwischen dem aktuell erfassten Absorptionsspektrum der Flüssigkeitsprobe und einem Referenz-Absorptionsspektrum kann somit zur Klassifizierung des Messwerts des Parameters hinsichtlich seiner Messwert-Güte herangezogen werden: Beispielsweise kann bei starker Ähnlichkeit des aktuell gemessenen Absorptionsspektrums mit dem Referenz-Absorptionsspektrum der Referenzprobe, mit der die letzte Kalibrierung der spektrometrischen Sonde durchgeführt wurde, der Messwert als sehr verlässlich eingestuft werden. Bei geringer Ähnlichkeit ist der Messwert dagegen wenig verlässlich. Der mindestens eine Ähnlichkeitsparameter ist also ein Maß für die Verlässlichkeit des Messwerts, auch als Messwert-Güte bezeichnet. Die Messwert-Güte kann wiederum auch als Diagnoseparameter zur Sensordiagnose dienen, worauf weiter unten noch ausführlicher eingegangen wird.

Eine weitere Klassifizierung des Messwerts kann dahingehend erfolgen, dass die spektrometrische Vorrichtung automatisch erkennt, ob es sich bei dem Messwert um einen Testwert handelt, der mit einer bestimmten Referenzsubstanz als Flüssigkeitsprobe ermittelt wurde. Bei hoher Ähnlichkeit zwischen dem aktuell erfassten Absorptionsspektrum und dem Referenz-Absorptionsspektrum einer Referenzsubstanz kann die spektrometrische Vorrichtung automatisch, ohne weitere Eingaben durch eine Bedienperson, erkennen, dass es sich bei einer aktuellen Flüssigkeitsprobe um die Referenzsubstanz handelt, und den Messwert als Testwert klassifizieren.

Der Verfahrensschritt des Vergleichens des ermittelten Absorptionsspektrums im vorgegebenen Wellenlängenbereich mit mindestens einem Referenz-Absorptionsspektrum einer Referenzprobe bekannter Zusammensetzung kann folgende Schritte umfassen:
Ermitteln von Ist-Werten mindestens eines Satzes von vorgegebenen Prüfparametern aus dem ermittelten Absorptionsspektrum;
Vergleichen des ermittelten Satzes von Ist-Werten mit mindestens einem hinterlegten Satz, insbesondere mit mehreren hinterlegten Sätzen, von Soll-Werten der Prüfparameter für den ermittelten Messwert des Parameters der Flüssigkeitsprobe oder eines damit korrelierten Werts, wobei die Prüfparameter des Satzes von Prüfparametern aus dem Referenz-Absorptionsspektrum der Referenzprobe abgeleitet sind, und wobei die Prüfparameter derart vorgegeben sind, dass sie charakteristische Eigenschaften des Referenz-Absorptionsspektrums repräsentieren.

Der Vergleich von aus dem Absorptionsspektrum abgeleiteten Ist-Werten mit hinterlegten Soll-Werten von Prüfparametern dient zur Prüfung, in wie weit ein aktuell gemessenes Absorptionsspektrum mit einem Referenz-Absorptionsspektrum übereinstimmt. Je besser die Ist-Werte der Prüfparameter mit den hinterlegten Soll-Werten übereinstimmen, umso ähnlicher ist das aktuell gemessene Absorptionsspektrum zum Referenz-Absorptionsspektrum. Die Prüfparameter werden beispielsweise so vorgegeben, dass sie charakteristische Merkmale des Referenz-Absorptionsspektrums repräsentieren. Der mit dem Messwert korrelierte Wert kann beispielsweise eine Konzentration sein.

Die Sätze von Soll-Werten der Prüfparameter können beispielsweise in einem Datenspeicher der spektrometrischen Vorrichtung, insbesondere in einem Datenspeicher einer Steuereinheit der spektrometrischen Vorrichtung, hinterlegt sein. Die Steuereinheit kann neben dem Datenspeicher einen Mikroprozessor umfassen, der zur Verarbeitung der Messdaten und zur Auswertung der Messergebnisse geeignet ist. Mit Hilfe der Steuereinheit der spektrometrischen Vorrichtung kann das hier und im Folgenden beschriebene Verfahren vollautomatisch durchgeführt werden.

Der Verfahrensschritt des Bestimmens mindestens eines Ähnlichkeitsparameters anhand des Vergleichs des ermittelten Absorptionsspektrums im vorgegebenen Wellenlängenbereich mit mindestens einem Referenz-Absorptionsspektrum mindestens einer Referenzprobe bekannter Zusammensetzung kann folgende Schritte umfassen:
Bestimmen von Ähnlichkeitsparametern aus einem Vergleich der Ist-Werte für die Prüfparameter mit den entsprechenden hinterlegten Soll-Werten der Prüfparameter. Zu jedem Paar von Ist-Wert und
Soll-Wert jeweils eines Prüfparameters kann also jeweils ein Ähnlichkeitsparameter ermittelt werden.

Das Ermitteln des Absorptionsspektrums kann die Schritte umfassen:
Durchstrahlen eines die Flüssigkeitsprobe enthaltenden Volumens mit Messstrahlung mindestens des vorgegebenen Wellenlängenbereichs entlang eines Messpfades zwischen einer Lichtquelle und
einem Empfänger, wobei die Messstrahlung durch einen ersten Fensterabschnitt in das Volumen eintritt und durch einen zweiten Fensterabschnitt aus dem Volumen austritt;
Erfassen der Intensität der durch den zweiten Fensterabschnitt ausgetretenen Messstrahlung mittels des Empfängers; und
Ermitteln der Intensität oder einer daraus abgeleiteten Größe, insbesondere der Absorption der Messstrahlung entlang des Messpfades, als Funktion der Messwellenlänge.

Das Ermitteln des Messwerts des Parameters der Flüssigkeitsprobe kann die Schritte umfassen: Integrieren des Absorptionsspektrums im vorgegebenen Wellenlängenbereich;
Anhand einer hinterlegten, aus Kalibriermessungen gewonnenen Zuordnungsvorschrift Ermitteln des Messwerts oder eines damit korrelierten Wertes, insbesondere einer Konzentration, aus dem Ergebnis der Integration.

Die Integration des Absorptionsspektrums im vorgegebenen Wellenlängenbereich kann nach aus dem Stand der Technik bekannten Verfahren erfolgen, insbesondere unter Berücksichtigung einer Grundlinienkorrektur (sog. baseline-Korrektur), bei der Beiträge zum Absorptionsspektrum, die nicht durch Absorption der Messstrahlung durch in der Flüssigkeitsprobe enthaltene Spezies zustande kommen, zunächst abgezogen werden. Solche Beiträge können beispielsweise durch Referenzmessungen mittels Referenzabsorbern ermittelt werden. Ein derartiges Verfahren und eine entsprechend zur Durchführung des Verfahrens geeignete Vorrichtung ist in der noch nicht veröffentlichten deutschen Patentanmeldung DE 102009002570.7 der Anmelderin beschrieben.

Die Zuordnungsvorschrift kann beispielsweise eine Kalibrierfunktion sein. Die zugehörigen Kalibrierparameter, z.B. im Falle einer linearen Kalibrierfunktion Nullpunkt bzw. Offset und Steilheit, werden durch einen Vergleich eines an einer Referenzprobe ermittelten Messwerts mit dem in einem Laborverfahren bestimmten Messwert des Parameters, z.B. des CSB- oder TOC-Werts der Referenzprobe, ermittelt. Dieser Vorgang wird auch als Kalibrierung bzw. als Justierung bezeichnet.

Als Prüfparameter werden vorzugsweise solche Parameter ausgewählt, die für das Referenz-Absorptionsspektrum charakteristisch sind, z.B. eine Wellenlänge eines Maximums, eine Wellenlänge eines Minimums, einen Absorptionswert oder einen Intensitätswert eines Maximums, einen Absorptionswert oder einen Intensitätswert eines Minimums, einen Wellenlängenbereich, in dem die Absorption einer Basislinie des Absorptionsspektrums entspricht, eine erste Ableitung in einem vorgegebenen Punkt des Absorptionsspektrums, eine Differenz oder ein Verhältnis von Absorptions- oder Intensitätswerten bei ausgewählten Wellenlängen des Referenz-Absorptionsspektrums, z.B. von Absorptionswerten oder Intensitätswerten von absoluten oder lokalen Minima oder Maxima oder von Intervallgrenzen bestimmter Wellenlängenbereiche.

Dabei werden für einen Satz von Prüfparametern vorzugsweise sowohl solche Prüfparameter ausgewählt, die sich in Abhängigkeit von der Konzentration der Referenzprobe im wesentlichen nicht ändern, wie z.B. die Wellenlängen eines Maximums oder eines Minimums oder das Verhältnis von Absorptionswerten oder Intensitätswerten, als auch solche Prüfparameter, die sich in Abhängigkeit von der Konzentration der Referenzprobe ändern, wie z.B. die Intensitäts- oder Absorptionswerte eines Maximums oder eines Minimums oder eine Differenz von Absorptionswerten oder Intensitätswerten.

In einer Verfahrensvariante sind im Speicher der Vorrichtung unterschiedliche Sätze von Prüfparametern hinterlegt, die charakteristische Merkmale von Referenz-Absorptionsspektren unterschiedlicher Referenzproben repräsentieren. Dabei können die aus dem aktuell gemessenen Absorptionsspektrum abgeleiteten Ist-Werte der Prüfparameter mit einigen oder allen unterschiedlichen Sätzen von Prüfparametern verglichen werden.

Der Soll-Wert eines Prüfparameters kann in Form einer Kriterienfunktion hinterlegt sein, wobei die Kriterienfunktion den Prüfparameter als Funktion des Parameters der Flüssigkeitsprobe oder einer daraus abgeleiteten Konzentration angibt, und wobei als Soll-Wert der Funktionswert der Kriterienfunktion bei dem als Messwert ermittelten Wert des Parameters der Flüssigkeitsprobe oder der daraus abgeleiteten Konzentration verwendet wird.

Die Ähnlichkeitsparameter können die Abweichung des Ist-Werts vom zugehörigen Soll-Wert in absoluten Zahlenwerten angeben. Es ist auch möglich, dass zusätzlich zu den Soll-Werten jeweils ein Toleranzintervall hinterlegt ist, und die Ähnlichkeitsparameter als prozentuale Abweichung des Ist-Werts vom Soll-Wert bezogen auf das Toleranzintervall bestimmt werden. Sind die Soll-Werte in Form von Kriterienfunktionen hinterlegt, können die Grenzen des Toleranzintervalls durch eine obere und eine untere Toleranzfunktion definiert werden. Die konzentrations- bzw. parameterabhängige Kriterienfunktion und die zugehörigen Toleranzfunktionen stellen in diesem Fall, begrenzt durch die untere und obere Messgrenze des Messverfahrens, das gesamte Prüffeld der jeweiligen Kriterienfunktion dar.

In einer Weiterbildung des Verfahrens werden aus den ermittelten Ähnlichkeitsparametern Gütewerte abgeleitet, aus denen auf die Messgüte des Messwerts geschlossen wird, wobei insbesondere aus den Gütewerten ein Bewertungsparameter ermittelt wird.

Für die Gütewerte können Wertebereiche vorgegeben sein, anhand derer der Messwert hinsichtlich seiner Messgüte klassifiziert wird, wobei insbesondere Schwellenwerte und/oder Wertebereiche für die Gütewerte oder den Bewertungsparameter vorgegeben sind, anhand derer der Messwert hinsichtlich seiner Messgüte klassifiziert wird. Die Klassifizierung der Messgüte gibt Aufschluss über die Notwendigkeit einer Kalibrierung bzw. Justierung der spektrometrischen Vorrichtung. Die Kalibrierung bzw. Justierung kann beispielsweise anhand einer Referenzierung des gemessenen Absorptionsspektrums auf einen im Labor durch Analyse einer Flüssigkeitsprobe ermittelten Wert des zu bestimmenden Parameters erfolgen.

Es ist möglich, die Gütewerte und/oder den Bewertungsparameter zusammen mit dem Messwert des Güteparameters auszugeben. Auf diese Weise kann sich eine Bedienperson ein Bild davon machen, wie verlässlich der aktuell ausgegebene Messwert ist.

Die Gütewerte und/oder der daraus ermittelte Bewertungsparameter können zur Sensordiagnose bzw. zur Diagnose der spektrometrischen Vorrichtung verwendet werden. Beispielsweise kann anhand der Gütewerte und/oder des Bewertungsparameters abgeleitet werden, ob eine Neukalibrierung bzw. eine Neujustierung der spektrometrischen Vorrichtung erforderlich ist. Weiterhin kann abgeleitet werden, ob Wartungsmaßnahmen, wie z.B. die Reinigung oder der Austausch von Teilen der spektrometrischen Vorrichtung, durchgeführt werden müssen. Ergibt die Sensordiagnose, dass eine Kalibrierung bzw. Justierung oder eine Wartungsmaßnahme erforderlich ist, kann eine Warnmeldung ausgegeben werden. Es ist auch möglich, dass die Vorrichtung aufgrund des Ergebnisses der Sensordiagnose selbständig, d.h. automatisch, eine Kalibrierung bzw. Justierung oder Wartungsmaßnahmen auslöst, wie z.B. die Reinigung von Messfenstern oder die Spülung von Bauteilen, z.B. eines Probenraums, mit einer Reinigungsflüssigkeit.

Die Ähnlichkeitsparameter, die Gütewerte und/oder die Bewertungsparameter können über eine Vielzahl von aufeinander folgenden Messungen an einer Vielzahl von Flüssigkeitsproben langfristig erfasst und statistisch ausgewertet werden. Eine prädiktive Diagnostik kann realisiert werden, indem anhand der Historie der erfassten und gespeicherten Ähnlichkeitsparameter, Gütewerte und/oder Bewertungsparameter ein Zeitpunkt in der Zukunft ermittelt wird, zu dem eine Wartung oder eine Kalibrierung bzw. Justierung erforderlich sein wird. Dieser Zeitpunkt kann ausgegeben werden, so dass eine Bedienperson die Wartungsmaßnahme oder Justierung bzw. Kalibrierung einplanen kann. Die Ermittlung des Zeitpunkts für eine Wartungsmaßnahme oder Justierung bzw. Kalibrierung kann beispielsweise erfolgen, indem der zeitliche Verlauf der genannten Werte extrapoliert wird, und daraus bestimmt wird, wann ein vorgegebener Schwellenwert erreicht werden wird. Der Schwellenwert kann eine nicht mehr tolerierbare Abweichung des gemessenen Absorptionsspektrums der Flüssigkeitsprobe von dem Referenz-Absorptionsspektrum bzw. eine nicht mehr akzeptable Messgüte repräsentieren.

In verschiedenen Verfahrensvarianten kann das Vergleichen des ermittelten Absorptionsspektrums im vorgegebenen Wellenlängenbereich
entweder mit mindestens einem Referenz-Absorptionsspektrum einer Referenzsubstanz,
oder mit einem Referenz-Absorptionsspektrum einer Referenzprobe bekannter Zusammensetzung, beispielsweise einer Abwasserprobe,
oder mit Referenz-Absorptionsspektren mehrerer Referenzproben unterschiedlicher Zusammensetzung, beispielsweise unterschiedlich zusammengesetzter Abwasserproben, durchgeführt werden.

Aus dem Vergleich mit dem Referenz-Absorptionsspektrum einer Referenzsubstanz kann bei einer hohen Ähnlichkeit darauf geschlossen werden, dass es sich bei der Flüssigkeitsprobe um die Referenzsubstanz handelt. Dies kann beispielsweise dazu genutzt werden, Testroutinen automatisch von der Vorrichtung durchführen zu lassen. Wenn die Vorrichtung erkennt, dass es sich bei der aktuellen Flüssigkeitsprobe um eine bestimmte Referenzsubstanz handelt, kann sie automatisch eine Testroutine starten, ohne dass eine Eingabe durch eine Bedienperson erforderlich ist.

Der Vergleich mit einem Referenz-Absorptionsspektrum einer Referenzprobe bekannter Zusammensetzung kann dazu genutzt werden, die Ähnlichkeit zwischen dem aktuell gemessenen Absorptionsspektrum der Flüssigkeitsprobe und dem Referenz-Absorptionsspektrum der zuletzt zur Kalibrierung bzw. Justierung verwendeten Referenzprobe zu überprüfen. Wenn das aktuelle Absorptionsspektrum zu stark von dem Referenz-Absorptionsspektrum abweicht, lässt sich darauf schließen, dass der mit den aktuell verwendeten Kalibrierparametern bestimmte Messwert möglicherweise nicht mehr ausreichend verlässlich ist.

Der Vergleich mit Referenz-Absorptionsspektren mehrerer Referenzproben unterschiedlicher Zusammensetzung erlaubt es, diejenige Referenzprobe zu ermitteln, deren Absorptionsspektrum die größte Ähnlichkeit mit der aktuellen Flüssigkeitsprobe aufweist.

In dieser zuletzt genannten Verfahrensvariante wird ein Vergleich des Absorptionsspektrums der Flüssigkeitsprobe im vorgegebenen Wellenlängenbereich mit Referenz-Absorptionsspektren mehrerer Referenzproben unterschiedlicher Zusammensetzung durchgeführt, wobei dasjenige Referenz-Absorptionsspektrum ermittelt wird, das dem Absorptionsspektrum der Flüssigkeitsprobe am ähnlichsten ist. Dabei handelt es sich um dasjenige Referenz-Absorptionsspektrum, bei dem der Vergleich von Soll-Werten mit Ist-Werten der hinterlegten Prüfparameter die geringste durch die Ähnlichkeitsparameter repräsentierte Abweichung ergibt.

Hierzu werden beispielsweise die Ist-Werte der vorgegebenen Prüfparameter mit mehreren hinterlegten Sätzen von Soll-Werten der Prüfparameter verglichen, und derjenige Satz von Soll-Werten der Prüfparameter ermittelt, bei dem die Abweichung der Ist-Werte von den hinterlegten Soll-Werten am geringsten ist. Das Referenz-Absorptionsspektrum, das diesem Satz von Soll-Werten bzw. Prüfparametern zugrunde liegt, ist entsprechend dasjenige Referenz-Absorptionsspektrum, das die größte Ähnlichkeit mit dem aktuell erfassten Absorptionsspektrum der Flüssigkeitsprobe aufweist. In der Regel weist die Zusammensetzung der zu diesem Referenz-Absorptionsspektrum gehörigen Referenzprobe eine große Ähnlichkeit mit der Zusammensetzung der aktuellen Flüssigkeitsprobe auf, oder ist, bei besonders großer Ähnlichkeit aller Soll- und Ist-Werte, identisch mit der Referenzprobe.

Diese Verfahrensvariante kann insbesondere genutzt werden, um über die durch die weiter oben beschriebenen Gütewerte mögliche Feststellung hinaus, dass die Verlässlichkeit der von der Vorrichtung in einer Serie von online-Messungen an einer Reihe von Flüssigkeitsproben ermittelten Messwerte abnimmt, zusätzlich einen Anhaltspunkt dafür zu bekommen, welche Art von Veränderungen in der Flüssigkeitsprobe gegenüber vorher untersuchten Flüssigkeitsproben aufgetreten sind. Wird durch den Vergleich des Absorptionsspektrums der Flüssigkeitsprobe im vorgegebenen Wellenlängenbereich mit Referenz-Absorptionsspektren mehrerer Referenzproben festgestellt, dass nicht mehr das Referenz-Absorptionsspektrum der zur letzten Kalibrierung herangezogenen Referenzprobe, beispielsweise einer ersten Abwasser-Zusammensetzung, die größte Ähnlichkeit zu der aktuellen Flüssigkeitsprobe aufweist, sondern das Referenz-Absorptionsspektrum einer anderen Referenzprobe, beispielsweise einer zweiten Abwasser-Zusammensetzung, so kann daraus in der Regel geschlossen werden, dass die Zusammensetzung der Flüssigkeitsprobe ähnlich der zweiten anderen Referenzprobe, im Beispiel der Abwasser-Zusammensetzung, ist.

Vorzugsweise wird eine Angabe zu der Referenzprobe, deren Referenz-Absorptionsspektrum im vorgegebenen Wellenlängenbereich die größte Ähnlichkeit mit dem Absorptionspektrum der Flüssigkeitsprobe aufweist, mit dem Messwert ausgegeben. Eine solche Angabe kann beispielsweise eine Bezeichnung der Referenzprobe sein.

In einer Weiterbildung dieser Verfahrensvariante können zu jeder Referenzprobe aus Referenzmessungen mit der Referenzprobe gewonnene Kalibrierparameter und/oder eine Kalibrierfunktion hinterlegt sein, wobei ein korrigierter Messwert des Parameters der Flüssigkeitsprobe unter Verwendung der zu der Referenzprobe hinterlegten Kalibrierparameter bestimmt wird, deren Referenz-Absorptionsspektrum dem ermittelten Absorptionsspektrum der Flüssigkeitsprobe am ähnlichsten ist. Vorzugsweise sind die Kalibrierparameter und/oder die Kalibrierfunktion zusammen oder verknüpft mit dem Satz von Prüfparametern bzw. Kriterienfunktionen, die die charakteristischen Merkmale des jeweiligen Absorptionsspektrums der Referenzprobe repräsentieren, im Speicher der spektrometrischen Vorrichtung hinterlegt.

Im voranstehend beschriebenen Beispielfall, dass sich bei einer Serie von Bestimmungen eines Messwerts an mehreren nacheinander untersuchten Flüssigkeitsproben die Zusammensetzung der Flüssigkeitsproben von einer ersten Abwasserzusammensetzung zu einer zweiten Abwasserzusammensetzung ändert, kann auf diese Weise der stark fehlerbehaftete, mit Kalibrierdaten, die aus Referenzmessungen mit Referenzproben der ersten Abwasserzusammensetzung gewonnen wurden, ermittelte Messwert durch einen korrigierten Messwert ersetzt werden, der mittels Kalibrierdaten, die aus Referenzmessungen mit Referenzproben der zweiten Abwasserzusammensetzung gewonnen wurden, bestimmt wird.

In einer anderen Weiterbildung dieser Verfahrensvariante werden zu dem ermittelten Referenz-Absorptionsspektrum hinterlegte Kriterienfunktionen, welche Verläufe von vorgegebenen Prüfparametern als Funktion des Parameters der Flüssigkeitsprobe oder einer daraus abgeleiteten Konzentration angeben, variiert,
bis ein oder mehrere Ähnlichkeitsparameter, die aus einem Vergleich von einem oder mehreren Soll-Werten der vorgegebenen Prüfparameter, mit einem oder mehreren Ist-Werten der vorgegebenen Prüfparameter ermittelt werden, maximal sind,
wobei die Soll-Werte für den aus dem Absorptionsspektrum der Flüssigkeitsprobe ermittelten Messwert des Parameters der Flüssigkeitsprobe oder für einen damit korrelierten Wert aus den Kriterienfunktionen abgeleitet werden, und
wobei die Ist-Werte aus dem Absorptionsspektrum der Flüssigkeitsprobe bestimmt werden.

Diese Verfahrensvariante ist insbesondere dann vorteilhaft, wenn sich das Absorptionsspektrum der aktuellen Abwasserzusammensetzung von allen hinterlegten Referenz-Absorptionsspektren für verschiedene Abwasserzusammensetzungen stark unterscheidet. In diesem Fall können zwar nach einer Neukalibrierung bzw. Neujustierung der spektrometrischen Vorrichtung durch Referenzierung des gemessenen Absorptionsspektrums auf einen im Labor durch Analyse einer Flüssigkeitsprobe ermittelten Wert des zu bestimmenden Parameters wieder verlässliche Messwerte erhalten werden. Wenn jedoch keines der hinterlegten Referenz-Absorptionsspektren den Absorptionsspektren der aktuell zu untersuchenden Flüssigkeitsproben ausreichend ähnelt, spiegeln die entsprechend ermittelten Ähnlichkeitsparameter bzw. die daraus ermittelten Gütewerte oder Bewertungsparameter jedoch diese Verlässlichkeit nicht wieder. Dies kann beispielsweise dazu führen, dass eine Warnmeldung ausgegeben wird, obwohl die Messgüte noch ausreichend ist. Durch die Anpassung der Kriterienfunktionen wird dies vermieden. Diese Verfahrensvariante erlaubt auch eine automatische Feldjustierung.

Ausführungsbeispiele der Erfindung werden nun im Detail anhand der Figuren erläutert. Es zeigen:

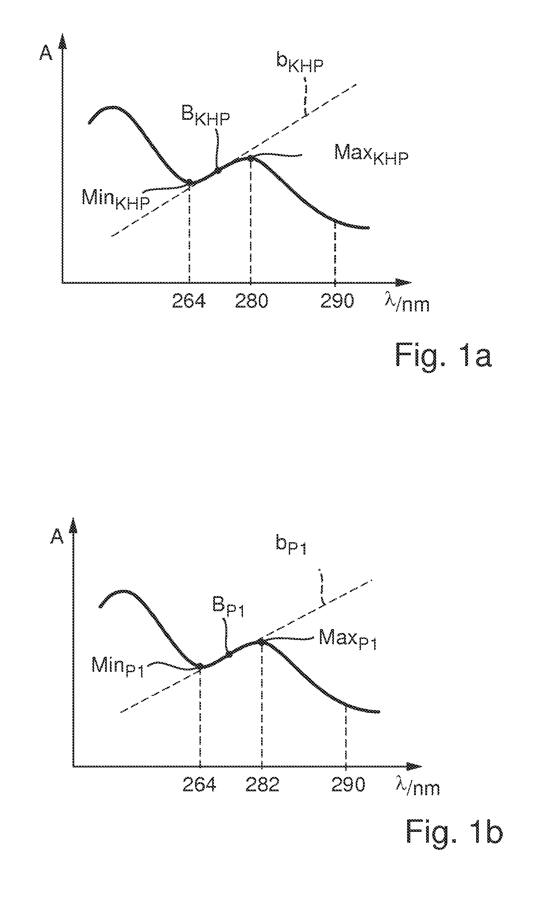

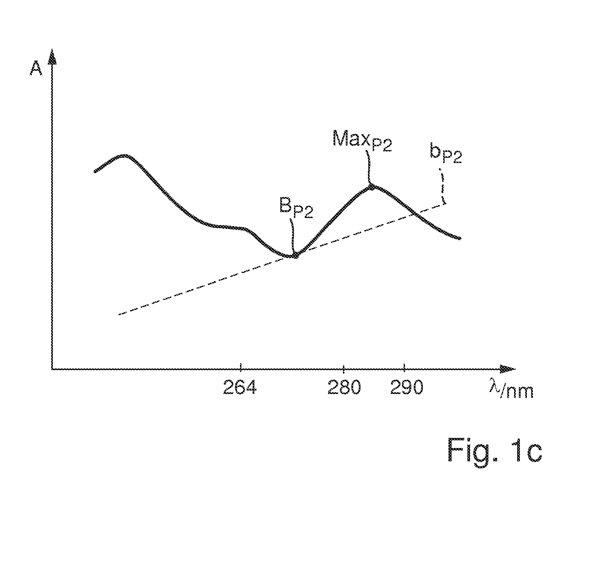

- Fig. 1: a) eine schematische Darstellung eines Absorptionsspektrums einer Lösung von Kaliumhydrogenphthalat in Wasser; b) eine schematische Darstellung eines Absorptionsspektrums einer ersten Flüssigkeitsprobe; c) eine schematische Darstellung eines Absorptionsspektrums einer zweiten Flüssigkeitsprobe;

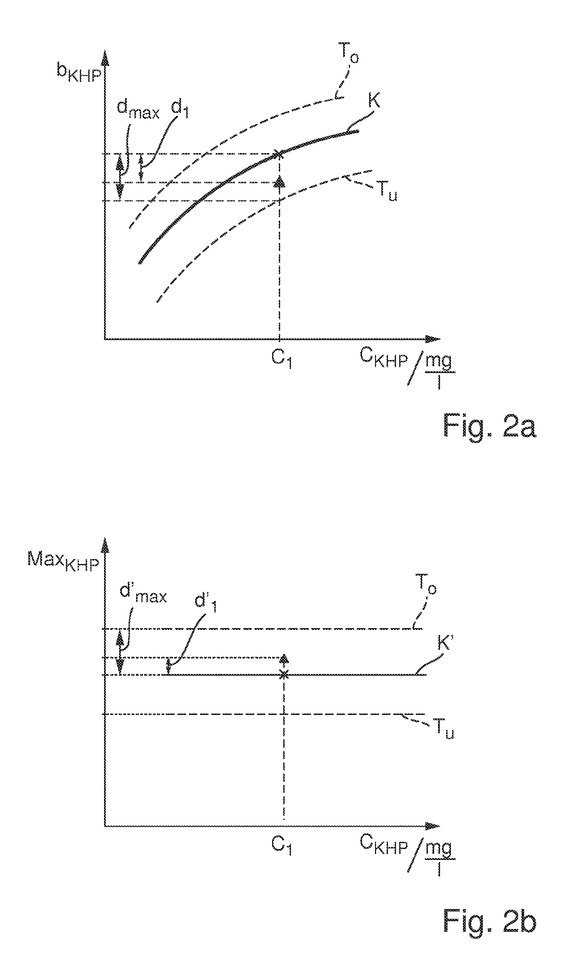

- Fig. 2: a) ein Diagramm eines ersten Prüfparameters als Funktion der Konzentration zum Vergleich mit einem aus dem in Fig. 1 b) dargestellten Absorptionsspektrum ermittelten Ist-Wert; b) ein Diagramm eines zweiten Prüfparameters als Funktion der Konzentration zum Vergleich mit einem aus dem in Fig. 1 b) dargestellten Absorptionsspektrum ermittelten Ist-Wert;

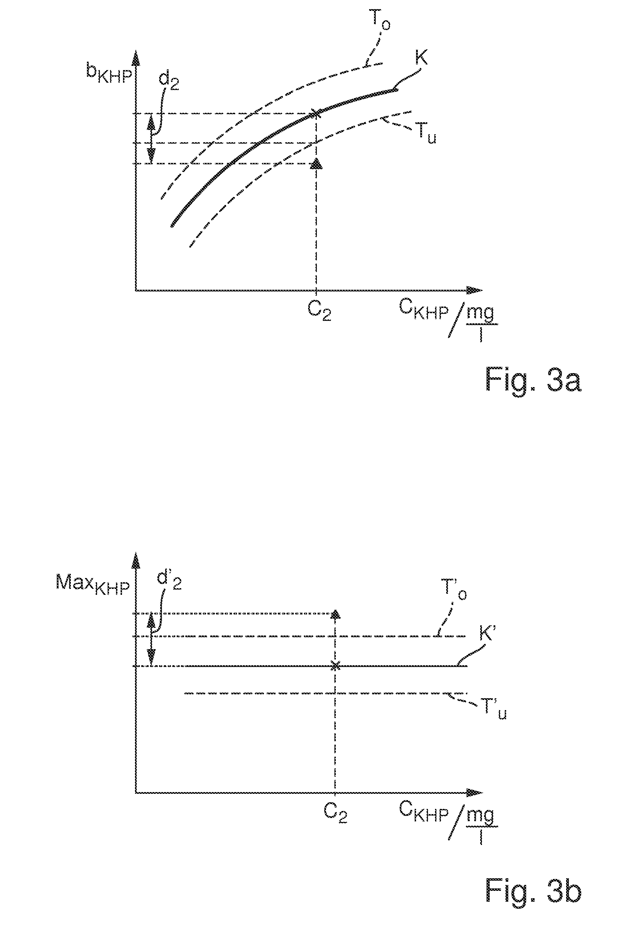

- Fig. 3: c) ein Diagramm eines ersten Prüfparameters als Funktion der Konzentration zum Vergleich mit einem aus dem in Fig. 1 c) dargestellten Absorptionsspektrum ermittelten Ist-Wert; d) ein Diagramm eines zweiten Prüfparameters als Funktion der Konzentration zum Vergleich mit einem aus dem in Fig. 1 c) dargestellten Absorptionsspektrum ermittelten Ist-Wert;

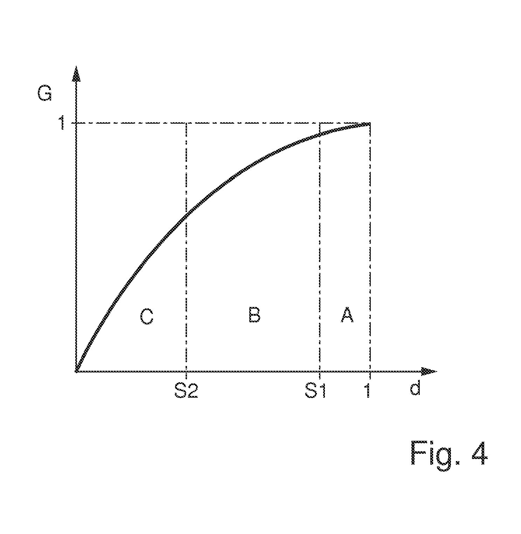

- Fig. 4: ein den Zusammenhang zwischen einem Ähnlichkeitsparameter und einem daraus abgeleiteten Güteparameter schematisch darstellendes Diagramm.

Zunächst wird exemplarisch anhand der in Fig. 1 a) und b) dargestellten Absorptionsspektren ein Verfahren dargestellt, mit dem ein Absorptionsspektrum einer Flüssigkeitsprobe unbekannter Zusammensetzung mit einem Referenz-Absorptionsspektrum einer Referenz-Flüssigkeitsprobe verglichen werden kann.

In Fig. 1 a) ist ein Absorptionsspektrum von Kaliumhydrogenphthalat (KHP) im Wellenlängenbereich zwischen 200 und 300 nm skizziert. KHP wird häufig als Referenzsubstanz für spektrometrische Vorrichtungen zur Bestimmung eines Summenparameters wie des CSB-Werts oder eines TOC-Werts einer Flüssigkeitsprobe verwendet. Das Absorptionsspektrum von KHP besitzt einige charakteristische Merkmale, beispielsweise ein Absorptionsminimum Min_{KHP} bei 264 nm und ein Absorptionsmaximum Max_{KHP} bei 280 nm. Ein weiteres charakteristisches Merkmal ist die Steigung b_{KHP} einer Gerade, welche im Wendepunkt B_{KHP} der zwischen dem Absorptionsminimum Min_{KHP} und dem Absorptionsmaximum Max_{KHP} ansteigenden Flanke des zum Absorptionsmaximum Max_{KHP} gehörenden Peaks an das Absorptionsspektrum anliegt.

In Fig. 1 b) ist ein Absorptionsspektrum einer ersten Flüssigkeitsprobe zunächst unbekannter Zusammensetzung skizziert. Um das Absorptionsspektrum einer unbekannten Flüssigkeitsprobe mit dem Referez-Absorptionsspektrum von KHP als Referenzsubstanz zu vergleichen, können aus dem Referenz-Absorptionsspektrum von KHP Prüfparameter ermittelt werden, die charakteristische Merkmale des Absorptionsspektrums, wie beispielsweise die Lage der Extrema oder die Steigung einer Peakflanke, repräsentieren. Im vorliegenden Beispiel werden als vorgegebene Prüfparameter zur Prüfung der Ähnlichkeit zwischen dem Absorptionsspektrum der unbekannten Flüssigkeitsprobe und dem Referenz-Absorptionsspektrum der als Referenzsubstanz dienenden KHP-Lösung die Steigung B_{KHP} einer im Wendepunkt B_{KHP} an die ansteigende Peakflanke anliegenden Geraden und die Lage, d.h. die Wellenlänge λ, des Absorptionsmaximums Max_{KHP} ausgewählt und vorgegeben. Zur Erhöhung der Sicherheit des im folgenden beschriebenen Verfahrens können weitere Prüfparameter herangezogen werden, wie beispielsweise die Wellenlänge λ des Absorptionsminimums Min_{KHP}, die Steigungen weiterer Peakflanken, die Absorptionswerte der Extrema Min_{KHP} und Max_{KHP}, und weitere Parameter. Vorzugsweise werden als Prüfparameter sowohl solche Merkmale ausgewählt, die konzentrationsunabhängig sind, als auch solche, die konzentrationsabhängig sind.

Um die Ähnlichkeit zwischen dem in Fig. 1 b) gezeigten Absorptionsspektrum der unbekannten Flüssigkeitsprobe zu dem in Fig. 1 a) gezeigten Referenz-Absorptionsspektrum von KHP zu quantifizieren, können aus dem Absorptionsspektrum der unbekannten Flüssigkeitsprobe ermittelte Ist-Werte der Prüfparameter mit aus dem Absorptionsspektrum von KHP ermittelten Soll-Werten der Prüfparameter verglichen werden, und aus dem Vergleich Ähnlichkeitsparameter, die die Abweichung der Ist- von den Soll-Werten repräsentieren, ermittelt werden. Dabei ist zu beachten, dass nur ein Teil der zuvor beispielhaft genannten, aus dem Referenz-Absorptionsspektrum von KHP ermittelten Prüfparameter konzentrationsunabhängig sind, wie beispielsweise die Wellenlängen der Extremwerte Max_{KHP} und Min_{KHP}. Andere Prüfparameter, wie beispielsweise der Betrag der Intensität bzw. der Absorption A der Extremwerte Max_{KHP} oder Min_{KHP} hängen jedoch wesentlich von der Konzentration der absorbierenden Substanz, hier KHP, ab. Um sowohl für die konzentrationsunabhängigen als auch für die konzentrationsabhängigen Prüfparameter einen Ähnlichkeitsvergleich zwischen dem Referenz-Absorptionsspektrum von KHP als Referenzsubstanz und dem Absorptionsspektrum der unbekannten Flüssigkeitsprobe durchführen zu können, werden deshalb zunächst sogenannte Kriterienfunktionen ermittelt, die die Beziehung zwischen dem jeweiligen Prüfparameter und der Konzentration der Referenzprobe angeben. Die Ermittlung solcher Kriterienfunktionen kann beispielsweise mit Hilfe einer Vielzahl von bei unterschiedlichen Konzentrationen der Referenzprobe aufgenommenen Absorptionsspektren erfolgen.

Zwei Beispiele für solche Kriterienfunktionen K und K' sind in Fig. 2 a) und b) dargestellt. In Fig. 2 a) ist als Beispiel für einen konzentrationsabhängigen Prüfparameter die Steigung B_{KHP} einer an die zwischen den Extremwerten Min_{KHP} und Max_{KHP} in einem Wendepunkt B_{KHP} an die ansteigende Flanke des Absorptionspeaks angelegten Geraden gegen die KHP-Konzentration aufgetragen. In Fig. 2 b) ist als Beispiel für einen konzentrationsunabhängigen Prüfparameter die Wellenlänge λ des Absorptionsmaximums Max_{KHP} als Funktion der Konzentration aufgetragen.

Um die Ähnlichkeit zwischen dem Absorptionsspektrum der unbekannten Flüssigkeitsprobe und einem Referenz-Absorptionsspektrum von KHP als Referenzprobe bei vergleichbarer Konzentration mit Hilfe der Kriterienfunktionen K und K' zu ermitteln, wird zunächst aus dem Absorptionsspektrum der unbekannten Flüssigkeitsprobe eine Konzentration abgeleitet. Dazu wird beispielsweise das Absorptionsspektrum über einen vorgegebenen Wellenlängenbereich integriert. Der Wellenlängenbereich wird so gewählt, dass signifikante Absorptionssignale der Referenzsubstanz, hier KHP, innerhalb dieses Wellenlängenbereiches liegen. Im vorliegenden Beispiel könnte der Wellenlängenbereich also zwischen 264 nm und 290 nm liegen. Aus dem ermittelten Integral wird anhand einer hinterlegten, aus Kalibriermessungen gewonnenen Zuordnungsvorschrift, beispielsweise einer Kalibrierfunktion, eine theoretische KHP-Konzentration c₁ abgeleitet. Dabei ist zu beachten, dass die Ableitung der theoretischen KHP-Konzentration im vorliegenden Beispiel eine rein rechnerische Maßnahme ist, um das Absorptionsspektrum der unbekannten Flüssigkeitsprobe mit dem Absorptionsspektrum von KHP als Referenzprobe vergleichen zu können. Die ermittelte KHP-Konzentration stellt in aller Regel keine "reale" in der Flüssigkeitsprobe enthaltene KHP-Konzentration dar. Nur wenn es sich bei der Flüssigkeitsprobe tatsächlich um eine KHP-Lösung handelt, ist die theoretische KHP-Konzentration auch die reale Konzentration von KHP in der Flüssigkeitsprobe.

Aus den in Fig. 2 a) und b) dargestellten Kriterienfunktionen K und K' kann nun der zur ermittelten theoretischen KHP-Konzentration c₁ zugehörige Soll-Wert der Prüfparameter b_{KHP} und Max_{KHP} abgelesen werden. Diese Soll-Werte sind in Fig. 2 a) und b) jeweils durch ein Kreuz markiert.

Aus dem Absorptionsspektrum der unbekannten Flüssigkeitsprobe lassen sich Ist-Werte der vorgegebenen Prüfparameter ermitteln. Dies ist in Fig. 1 b) dargestellt. Zur Ermittlung des Ist-Werts des Prüfparameters Max_{KHP} aus dem Absorptionsspektrum der Flüssigkeitsprobe wird im Wellenlängenbereich z.B. zwischen 264 nm und 290 nm nach einem Maximum des Absorptionsspektrums gesucht. Im gezeigten Beispiel beträgt die Wellenlänge des so gefundenen Absorptionsmaximums Max_{P1} der Flüssigkeitsprobe 282 nm. Zur Ermittlung des Ist-Werts des Prüfparameters b_{KHP} aus dem Absorptionsspektrum der Flüssigkeitsprobe wird an einem Punkt B_{P1} des Absorptionsspektrums der Flüssigkeitsprobe, dessen Wellenlänge (Abszissenwert) der Wellenlänge des Wendepunkts B_{KHP} der ansteigenden Flanke des Absorptionspeaks bei 280 nm im Referenz-Absorptionsspektrum von KHP entspricht, eine Gerade an das Absorptionsspektrum der Flüssigkeitsprobe angelegt. Bei der Festlegung des Abszissenwerts des Punktes B_{P1} kann die theoretische KHP-Konzentration c₁ mit berücksichtigt werden, da sich die Lage des Punktes B_{KHP} im KHP-Referenzspektrum konzentrationsabhängig verschieben kann. Die Steigung b_{P1} ist geringfügig flacher als die Steigung b_{KHP} der entsprechenden Geraden im Absorptionsspektrum von KHP in Fig. 1 a).

Die Ist-Werte sind in Fig. 2 a) und b) durch Dreiecke markiert. Die Ist-Werte können nun mit den als Kreuzen dargestellten Soll-Werten verglichen werden. Die aus dem Absorptionsspektrum der Flüssigkeitsprobe ermittelte Wellenlänge λ des Absorptionsmaximums Max_{P1} ist im vorliegenden Beispiel um 0,7% höher als die Wellenlänge λ des Absorptionsmaximums Max_{KHP} im Absorptionsspektrum von KHP. Die aus dem Absorptionsspektrum der Flüssigkeitsprobe ermittelte Geradensteigung b_{P1} ist im vorliegenden Beispiel etwas geringer als der entsprechende Soll-Wert b_{KHP} bei der ermittelten theoretischen KHP-Konzentration in Fig. 1 a).

Eine Quantifizierung der Abweichungen zwischen Ist- und Soll-Werten kann durch Bestimmung einer prozentualen Abweichung der Ist-Werte von den Soll-Werten vorgenommen werden. Die Abweichung kann insbesondere durch einen Ähnlichkeitsparameter repräsentiert werden, wobei zu jedem Paar von Ist- und Soll-Wert jeweils eines Prüfparameters ein Ähnlichkeitsparameter ermittelt wird.

Jedem Ähnlichkeitsparameter kann ein nach einer in einem Speicher der spektrometrischen Vorrichtung hinterlegten Zuordnungsvorschrift bestimmter Gütewert zugeordnet werden. In Fig. 4 ist beispielhaft der Zusammenhang eines derartigen Gütewerts G mit dem zugehörigen Ähnlichkeitsparameter d dargestellt. Besitzt der Ähnlichkeitsparameter d den Wert 1, besteht also Übereinstimmung zwischen dem Soll- und dem Ist-Wert, erreicht der Gütewert G ebenfalls den Maximalwert 1. Es kann nun ein erster Schwellenwert S1 für den Ähnlichkeitsparameter d festgelegt werden. Bei einem Ähnlichkeitsparameter d im Intervall A zwischen S1 und 1 ist der Gütewert G noch sehr nahe bei 1. Dies bedeutet zum einen, dass eine sehr hohe Übereinstimmung bzw. eine große Ähnlichkeit zwischen dem Soll- und dem Ist-Wert des betrachteten Prüfparameters besteht. Zum anderen ist die Güte des aus dem aktuellen Absorptionsspektrum bestimmten Messwerts der KHP-Konzentration in der Flüssigkeitsprobe sehr hoch, d.h. der Messwert ist sehr verlässlich.

Bei einem in einem Intervall B zwischen dem Schwellenwert S1 und einem vorgegebenen, niedrigeren zweiten Schwellenwert S2 liegenden Ähnlichkeitsparameterwert d ist der Gütewert G bereits erheblich niedriger als 1. Die Ähnlichkeit zwischen dem Soll- und dem Ist-Wert reicht demnach nicht aus, um mit Sicherheit eine Übereinstimmung zwischen Soll- und Ist-Wert des betrachteten Prüfparameters feststellen zu können. Entsprechend ist auch der Güteparameter G deutlich niedriger, d.h. der Messwert ist nicht so verlässlich wie bei einer besseren Übereinstimmung zwischen Soll- und Ist-Wert. Ein unterhalb des Schwellenwerts S2 im Intervall C liegender Ähnlichkeitsparameter d zeigt eine noch schlechtere Übereinstimmung zwischen Soll- und Ist-Wert an, was sich auch als eine noch geringere Güte des Messwerts äußert.

Im Folgenden werden zwei Beispiele für die Anwendung des hier beschriebenen Ähnlichkeitsvergleichs zwischen aus einem Absorptionsspektrum einer unbekannten Flüssigkeitsprobe abgeleiteten Ist-Werten für einen Satz von vorgegebenen Prüfparametern und Soll-Werten für den Satz von vorgegebenen Prüfparametern, die aus Absorptionsspektren einer Referenzprobe ermittelt worden sein können, beschrieben.

### Beispiel 1: Durchführung einer CSB-Bestimmung mittels einer spektrometrischen Vorrichtung mit automatischer Erkennung einer Testsubstanz

Die in diesem Beispiel verwendete spektrometrische Vorrichtung kann aufgebaut sein wie in EP 1 472 521 B1 oder der unveröffentlichten deutschen Patentanmeldung DE 102009002570.7 der Anmelderin beschrieben. Wie eingangs beschrieben, wird zunächst ein Absorptionsspektrum einer Flüssigkeitsprobe mindestens in einem vorgegebenen Wellenlängenbereich ermittelt. Die Bestimmung des chemischen Sauerstoffbedarfs der Flüssigkeitsprobe erfolgt zunächst durch Auswertung des Absorptionsspektrums in diesem vorgegebenen Wellenlängenbereich, beispielsweise durch Integration und Umrechnung des ermittelten Integrals mittels einer Zuordnungsvorschrift, insbesondere einer Kalibrierfunktion, in einen CSB-Wert oder einen damit korrelierten Wert, insbesondere eine Konzentration.

Beispielsweise bei Inbetriebnahme der spektrometrischen Vorrichtung kann sich die Notwendigkeit eines Tests der Vorrichtung ergeben. Dabei wird eine Referenzsubstanz bekannter Konzentration als Flüssigkeitsprobe vorgelegt, und deren CSB-Wert bestimmt. Dabei soll die Vorrichtung automatisch erkennen, dass gerade ein Test durchgeführt wird. Eine Voraussetzung hierfür ist, dass die Vorrichtung automatisch erkennt, dass es sich bei der vorgelegten Flüssigkeitsprobe um eine Referenzsubstanz handelt. Als Referenzsubstanz für einen derartigen Test kann beispielsweise eine wässrige Lösung von KHP verwendet werden.

In einem Datenspeicher der Vorrichtung ist ein Satz von Prüfparametern hinterlegt, die aus charakteristischen Merkmalen des Referenz-Absorptionsspektrums der Referenzsubstanz, hier der KHP-Lösung, ausgewählt wurden. Weiterhin ist im Datenspeicher der Vorrichtung ein entsprechender Satz von Kriterienfunktionen hinterlegt, die die Soll-Werte der Prüfparameter als Funktion der KHP-Konzentration angeben.

Aus einem neu gemessenen Absorptionsspektrum einer unbekannten Flüssigkeitsprobe wird zunächst nach dem anhand der Fig. 1 und 2 beschriebenen Vergleichsverfahren eine theoretische KHP-Konzentration c₁ der Flüssigkeitsprobe ermittelt, und daraus anhand der hinterlegten Kriterienfunktionen K und K' die Soll-Werte der Prüfparameter für die theoretische KHP-Konzentration c₁ abgeleitet. In Fig. 2 a) und b) sind die aus den Kriterienfunktionen K und K' bei der Konzentration c₁ ermittelten Soll-Werte jeweils als Kreuz dargestellt. Weiterhin ermittelt die spektrometrische Vorrichtung aus dem Absorptionsspektrum der unbekannten Flüssigkeitsprobe Ist-Werte der vorgegebenen Prüfparameter, die in Fig. 2 a) und b) als Dreiecke dargestellt sind.

In einem weiteren Schritt werden die Ist-Werte mit den Soll-Werten verglichen. Dieser Ähnlichkeitsvergleich zwischen den Ist- und den Soll-Werten kann durch Ermittlung einer prozentualen Abweichung erfolgen. Unterschreiten die prozentualen Abweichungen jeweils einen vorgegebenen Schwellenwert, d.h. liegen die Abweichungen der Ist-Werte von den Soll-Werten innerhalb eines durch den Schwellenwert definierten Toleranzintervalls, so wird die unbekannte Flüssigkeitsprobe als Referenzsubstanz KHP erkannt, und als solche klassifiziert.

Eine Vorgehensweise zur Quantifizierung der Abweichungen zwischen Soll- und Ist-Wert, die eine noch sicherere Erkennung der Flüssigkeitsprobe als Referenzsubstanz ermöglicht, besteht darin, die hinterlegten Soll-Werte mit einem Toleranzintervall zu versehen und die Ähnlichkeitsparameter bezogen auf das Toleranzintervall zu ermitteln. In Fig. 2 sind die obere und untere Grenze der Toleranzintervalle der Kriterienfunktionen K und K' in Fig. 2 a) und b) als gestrichelte Linien Tₒ und Tᵤ bzw. Tₒ' und Tᵤ' dargestellt. Die Ähnlichkeitsparameter d₁ bzw. d₁' werden als prozentuale Abweichung des Ist-Werts vom Soll-Wert bezogen auf das Toleranzintervall bestimmt. Lägen die Ist-Werte also genau auf der Kriterienfunktion bzw. fielen sie mit dem Soll-Wert zusammen, wären die Ähnlichkeitsparameter d₁ bzw. d₁' 100% bzw. 1. Lägen die Ist-Werte genau auf einer der Intervallgrenzen Tᵤ oder Tₒ oder außerhalb des Toleranzintervalls, hätten die Ähnlichkeitsparameter den Wert Null. Der Abstand d₁ des in Fig. 2 a) als Dreieck markierten Ist-Werts von dem als Kreuz markierten Soll-Wert entspricht etwa zwei Dritteln des Abstands dₘₐₓ zwischen dem Soll-Wert und dem unteren Toleranzwert bei der Konzentration c₁. Damit beträgt der Ähnlichkeitsparameter d₁ 0,33 bzw. 33%. In der Fig. 2 b) beträgt der Abstand d₁' des als Dreieck markierten Ist-Werts von dem als Kreuz markierten Soll-Wert etwa ein Drittel des Abstands dₘₐₓ' zwischen dem Soll-Wert und dem oberen Toleranzwert bei der Konzentration c₁. Damit beträgt der Ähnlichkeitsparameter d₁' 0,66 bzw. 66%. Auf diese Weise kann aus dem Vergleich des Ist-Werts mit dem für die ermittelte Konzentration c₁ aus der entsprechenden Kriterienfunktion abgeleiteten Soll-Wert jedes vorgegebenen Prüfparameters ein Satz von Ähnlichkeitsparametern ermittelt werden. Liegen alle Ähnlichkeitsparameter innerhalb der vorgegebenen Toleranzintervalle, wird die Flüssigkeitsprobe als KHP erkannt und klassifiziert.

In Fig. 1 c) ist ein weiteres Absorptionsspektrum einer zweiten unbekannten Flüssigkeitsprobe skizziert. Der Verlauf der Absorption als Funktion der Wellenlänge zeigt deutliche Abweichungen von den in Fig. 1 a) und Fig. 1 b) gezeigten Absorptionsspektren.

Aus dem in Fig. 1 c) gezeigten Absorptionsspektrum der zweiten unbekannten Flüssigkeitsprobe wird nach dem zuvor beschriebenen Verfahren zunächst wieder eine theoretische KHP-Konzentration c₂ der Flüssigkeitsprobe ermittelt, und daraus anhand der in Fig. 2 a) und b) dargestellten hinterlegten Kriterienfunktionen K und K' die Soll-Werte der Prüfparameter für die theoretische KHP-Konzentration c₂ abgeleitet. In Fig. 3 a) und b) sind die aus den Kriterienfunktionen K und K' bei der Konzentration c₂ ermittelten Soll-Werte jeweils als Kreuz dargestellt. Weiterhin werden aus dem in Fig. 1 c) dargestellten Absorptionsspektrum der unbekannten Flüssigkeitsprobe Ist-Werte der vorgegebenen Prüfparameter ermittelt.

Zur Ermittlung des Ist-Werts des Prüfparameters Max_{KHP} aus dem Absorptionsspektrum der Flüssigkeitsprobe wird im Wellenlängenbereich zwischen 264 nm und 290 nm nach einem Maximum des Absorptionsspektrums gesucht. Im vorliegenden Beispiel wird in diesem Wellenlängenbereich ein Maximum Max_{P2} gefunden. Dieses liegt bei einer Wellenlänge von etwa 287 nm. Zur Ermittlung des Ist-Werts des Prüfparameters b_{KHP} aus dem Absorptionsspektrum der Flüssigkeitsprobe wird an einem Punkt B_{P2} des Absorptionsspektrums der Flüssigkeitsprobe, dessen Wellenlänge (Abszissenwert) der Wellenlänge des Wendepunkts B_{KHP} der ansteigenden Flanke des Absorptionspeaks bei 280 nm im Referenz-Absorptionsspektrum von KHP entspricht, eine Gerade an das Absorptionsspektrum der Flüssigkeitsprobe angelegt. Wiederum kann bei der Festlegung des Abszissenwerts des Punktes B_{P1} die theoretische KHP-Konzentration c₂ mit berücksichtigt werden, da sich die Lage des Punktes B_{KHP} im KHP-Referenzspektrum konzentrationsabhängig verschieben kann. Die Steigung b_{P2} dieser Gerade ist deutlich geringer als die Steigung b_{KHP} der entsprechenden Gerade im Referenz-Absorptionsspektrum von KHP in Fig. 1 a).

Wie zuvor für das in Fig. 1 b) dargestellte Absorptionsspektrum beschrieben, werden in einem weiteren Schritt die Ist-Werte mit den Soll-Werten verglichen. Beim Absorptionsspektrum der Fig. 1 c) liegen die als Dreiecke dargestellten Ist-Werte für beide Prüfparameter b_{KHP} und Max_{KHP} außerhalb des durch die Intervallgrenzen Tᵤ und Tₒ vorgegebenen Toleranzintervalls. In beiden Fällen hat der Ähnlichkeitsparameter d₂ bzw. d₂' somit den Wert Null. Da bereits beide Ähnlichkeitsparameter außerhalb der vorgegebenen Toleranzintervalle liegen, wird, unabhängig von eventuell für andere Prüfparameter ermittelte Ähnlichkeitsparameter, die zweite Flüssigkeitsprobe nicht als KHP erkannt und entsprechend als "nicht KHP" klassifiziert.

Es ist auch möglich, den ermittelten Ähnlichkeitsparametern d₁, d₁', d₂, d₂' jeweils wie anhand von Fig. 4 beschrieben, einen Gütewert G zuzuordnen. Die Klassifizierung bzw. das Erkennen als KHP kann dann anhand des Gütewerts erfolgen. Beispielsweise kann, wenn alle Gütewerte bzw. Ähnlichkeitsparameter innerhalb des Intervalls A liegen, die Flüssigkeitsprobe als KHP eingestuft werden, während im Falle, dass nur ein Ähnlichkeitsparameter bzw. Gütewert außerhalb des Intervalls A oder gar im Intervall C liegt, die unbekannte Flüssigkeitsprobe als "nicht KHP" eingestuft wird.

### Beispiel 2: Klassifizieren des Messwerts des zu bestimmenden Parameters hinsichtlich der Messgüte und gegebenenfalls Anpassen der Kalibrierparameter

Wie im vorigen Beispiel 1 kann die in diesem Beispiel verwendete spektrometrische Vorrichtung aufgebaut sein wie in EP 1 472 521 B1 oder der unveröffentlichten deutschen Patentanmeldung DE 102009002570.7 der Anmelderin beschrieben. Wie eingangs beschrieben, wird mittels der spektrometrischen Vorrichtung ein Absorptionsspektrum einer Flüssigkeitsprobe mindestens in einem vorgegebenen Wellenlängenbereich ermittelt. Die Bestimmung des chemischen Sauerstoffbedarfs der Flüssigkeitsprobe erfolgt zunächst durch Auswertung des Absorptionsspektrum in diesem vorgegebenen Wellenlängenbereich, beispielsweise durch Integration und Umrechnen des ermittelten Integrals mittels einer Zuordnungsvorschrift, insbesondere einer Kalibriergeraden oder einer anderen Kalibrierfunktion, in einen CSB-Wert oder einen damit korrelierten Wert, insbesondere eine Konzentration. Die Zuordnungsvorschrift wird aus Kalibriermessungen, auch als Referenzmessungen bezeichnet, ermittelt, indem für eine oder mehrere Referenzproben des zu überwachenden Prozesses oder Gewässers, sowie gegebenenfalls für Verdünnungen der einen oder mehreren Referenzprobenproben, zunächst der CSB-Wert mittels chemischer Bestimmungsmethoden im Labor ermittelt wird, und die aus mit der spektrometrischen Vorrichtung erfassten Referenz-Absorptionsspektren derselben Referenzproben bzw. Verdünnungen der Referenzproben mit den im Labor bestimmten CSB-Werten verglichen werden.

Bei typischen online-Messungen der Summenparameter CSB (für den TOC-Wert gilt das gleiche) mit einer solchen spektrometrischen Vorrichtung werden in regelmäßigen zeitlichen Abständen aufeinander folgend Flüssigkeitsproben aus einem zu überwachenden Prozess oder einem zu überwachenden Gewässer entnommen und jeweils für jede Flüssigkeitsprobe ein Messwert des zu bestimmenden Parameters ermittelt. Im Laufe der Zeit kann sich die Zusammensetzung der Flüssigkeitsproben ändern, beispielsweise wenn in den zu überwachenden Prozess oder in das zu überwachende Gewässer eine zuvor nicht vorhandene organische Substanz eingeleitet wird. Durch die Änderung der Flüssigkeitszusammensetzung gegenüber der für die Kalibriermessungen verwendeten Referenzproben bzw. Verdünnungen der Referenzproben, ist die ursprünglich ermittelte Kalibrierfunktion nicht mehr optimal. Wird die ursprünglich ermittelte Kalibrierfunktion trotzdem zur Bestimmung es CSB-Werts auch der in ihrer Zusammensetzung veränderten Flüssigkeitsproben weiter verwendet, verschlechtert sich die Messgüte des CSB-Werts entsprechend.

Im Folgenden wird ein Verfahren beschrieben, mit dem gemessene CSB-Werte hinsichtlich ihrer Messgüte klassifiziert werden können.

Dabei wird aus einem neu gemessenen Absorptionsspektrum einer Flüssigkeitsprobe unbekannter Zusammensetzung analog wie bei dem vorstehend beschriebenen Verfahren zunächst ein CSB-Wert der Flüssigkeitsprobe oder ein damit korrelierter Wert, z.B. eine Konzentration, ermittelt. In einem Datenspeicher der Vorrichtung ist ein Satz von Prüfparametern und zugehörige Kriterienfunktionen der Soll-Werte für diese Prüfparameter hinterlegt, die charakteristische Eigenschaften des Referenz-Absorptionsspektrums der Referenzprobe bzw. der Referenzproben, mit denen die letzte Kalibrierung durchgeführt wurde, repräsentieren. Aus den hinterlegten Kriterienfunktionen werden die Soll-Werte der Prüfparameter für den ermittelten CSB-Wert bzw. den mit dem CSB-Wert korrelierten Wert, abgeleitet. Weiterhin werden aus dem Absorptionsspektrum der Flüssigkeitsprobe Ist-Werte der vorgegebenen Prüfparameter ermittelt. Diese Schritte erfolgen ganz analog wie bei dem anhand von Fig. 1 bis 3 für den Vergleich eines Absorptionsspektrums einer unbekannten Flüssigkeitsprobe mit einem KHP-Referenz-Absorptionsspektrum beschreibenen Verfahren, wobei jedoch die Referenzprobe in diesem Fall beispielsweise eine Abwasserprobe ist. Entsprechend können sich die aus dem Referenz-Absorptionsspektrum der Referenzprobe abgeleiteten Prüfparameter in diesem Fall von den aus dem KHP-Absoprtionsspektrum im voranstehenden Beispiel abgeleiteten Prüfparameter unterscheiden.

In einem weiteren Schritt werden die ermittelten Ist-Werte mit den Soll-Werten verglichen. Dieser Ähnlichkeitsvergleich zwischen den Ist- und den Soll-Werten kann z.B. durch Ermittlung einer prozentualen Abweichung erfolgen. Aus dem Ähnlichkeitsvergleich werden, wie weiter oben beschrieben, Ähnlichkeitsparameter ermittelt.

Jedem dieser Ähnlichkeitsparameter kann, wie anhand von Fig. 4 beschrieben, ein nach einer im Speicher hinterlegten Zuordnungsvorschrift bestimmter Gütewert zugeordnet werden. Mittels der vorgegebenen Schwellenwerte S1 und S2 lassen sich die Gütewerte beispielsweise in den Intervallen A, B und C entsprechende Klassen A, B, C einteilen, wobei die Klasse A eine sehr hohe Messwertgüte, die Klasse B eine mittlere Messwertgüte, und die Klasse C eine geringe Messwertgüte anzeigt. Anhand dieser Klassifizierung der einzelnen Gütewerte für die zu den einzelnen Prüfparametern ermittelten Ähnlichkeitsparameter kann eine Klassifizierung der Gesamt-Güte des aktuell ermittelten Messwerts des chemischen Sauerstoffbedarfs der Flüssigkeitsprobe erfolgen. Für die Gesamt-Güte können beispielsweise ebenfalls Klassen A, B und C vorgegeben werden. Im Falle, dass mindestens ein Gütewert in Klasse C vorliegt, kann die Gesamt-Güte des Messwerts ebenfalls mit Klasse C, d.h. "wenig verlässlich" eingestuft werden. Andere Zuordnungs-und Klassifizierungskriterien sind denkbar.

In einer Variante des Klassifizierungsverfahrens wird aus den Gütewerten ein Bewertungsparameter Δ bestimmt, beispielsweise durch Summierung, gewichtete Summierung oder durch eine andere Rechenvorschrift. Der Bewertungsparameter Δ ist zum einen ein Maß für die Ähnlichkeit des aktuell gemessenen Absorptionsspektrums der Flüssigkeitsprobe mit dem Absorptionsspektrum der Referenzprobe bzw. der Referenzproben, mit der bzw. mit denen die letzte Kalibrierung durchgeführt wurde. Damit ist der Bewertungsparameter Δ zum anderen auch ein Maß dafür, wie verlässlich der aktuell anhand der durch die letzte Kalibrierung gewonnenen Zuordnungsvorschrift bestimmte CSB-Messwert ist, da davon ausgegangen werden kann, dass der Messwert umso weniger verlässlich ist, je geringer die Ähnlichkeit zwischen dem Absorptionsspektrum der aktuellen Flüssigkeitsprobe und dem Referenz-Absorptionsspektrum der Referenzprobe ist.

Es kann ein Schwellenwert für den Bewertungsparameter Δ vorgegeben werden, bei dessen Überschreiten ein Alarm ausgegeben werden kann. Der Alarm weist darauf hin, dass eine erneute Kalibrierung hinsichtlich der Bestimmung des CSB-Werts durchgeführt werden muss, da die aktuell verwendeten Kalibrierparameter bzw. die aktuelle Kalibrierfunktion keine ausreichende Messgüte mehr gewährleisten.

In einer optionalen Weiterbildung dieses Verfahrens ist es möglich, bei Änderungen in der Zusammensetzung der zu überwachenden Prozessflüssigkeit oder des zu überwachenden Gewässers eine automatische Anpassung der Kalibrierparameter vorzunehmen.

Dabei wird nach dem vorstehend beschriebenen Verfahren ein Vergleich des für die aktuelle Flüssigkeitsprobe ermittelten Absorptionsspektrums im vorgegebenen Wellenlängenbereich mit Referenz-Absorptionsspektren mehrerer Referenzproben unterschiedlicher Zusammensetzung durchgeführt, und dasjenige Referenz-Absorptionsspektrum ermittelt, das dem Absorptionsspektrum der Flüssigkeitsprobe am ähnlichsten ist. Im Speicher der Vorrichtung sind zu jeder der möglichen Referenzproben Kalibrierparameter und/oder eine Kalibrierfunktion hinterlegt, die mittels Kalibriermessungen mit den Referenzproben zu einem früheren Zeitpunkt ermittelt wurden. Stellt sich beim Vergleich des Absorptionsspektrums der aktuellen Flüssigkeitsprobe heraus, dass nicht diejenige Referenzprobe, anhand derer die aktuell zur Messwertbestimmung verwendete Kalibrierfunktion bestimmt wurde, die der aktuellen Flüssigkeitsprobe ähnlichste ist, sondern dass das Referenz-Absorptionsspektrum einer anderen Referenzprobe dem aktuell ermittelten Absorptionsspektrum näher kommt, kann ein korrigierter Messwert ermittelt werden, indem die zur anderen Referenzprobe hinterlegten Kalibrierparameter und/oder die zur anderen Referenzprobe hinterlegte Kalibrierfunktion zur Messwertbestimmung herangezogen wird. Für weitere Messungen kann dann von vornherein die neue Kalibrierfunktion bzw. die neuen Kalibrierparameter verwendet werden.

Zu diesem Zweck können im Speicher der spektrometrischen Vorrichtung mehrere Sätze von Prüfparametern und zugehörige Kriterienfunktionen der Soll-Werte für diese Prüfparameter hinterlegt sein. Die unterschiedlichen Sätze von Prüfparametern und Kriterienfunktionen gehören jeweils zu den Referenz-Absorptionsspektren unterschiedlicher Referenzproben und repräsentieren charakteristische Eigenschaften dieser Referenz-Absorptionsspektren. Die unterschiedlichen Referenzproben können beispielsweise verschiedene Abwasserproben unterschiedlicher Zusammensetzung sein. Die Sätze von Prüfparametern können für alle Referenz-Absorptionsspektren dieselben Prüfparameter umfassen, sie können jedoch auch unterschiedliche Prüfparameter umfassen.

Aus dem Absorptionsspektrum der aktuellen Flüssigkeitsprobe werden Ist-Werte für alle hinterlegten Sätze von Prüfparametern ermittelt. Entsprechend werden anhand des aus dem Absorptionsspektrum der aktuellen Flüssigkeitsprobe ermittelten CSB-Werts oder des damit korrelierten Werts die entsprechenden Soll-Werte aus allen hinterlegten Sätzen von Kriterienfunktionen ermittelt. Nach einer der vorstehend beschriebenen Verfahrensvarianten werden durch Vergleich der entsprechenden Soll- und Ist-Werte Ähnlichkeitsparameter für jeden hinterlegten Satz von Prüfparametern bestimmt und somit Sätze von Ähnlichkeitsparametern gewonnen.

In einem weiteren Verfahrensschritt wird dann ermittelt, welcher Satz von Ähnlichkeitsparametern die geringsten Abweichungen zwischen Soll- und Ist-Werten der hinterlegten Prüfparametersätze repräsentiert. Der so ermittelte Satz von Prüfparametern repräsentiert entsprechend das Absorptionsspektrum derjenigen Referenzprobe, deren Zusammensetzung die größte Ähnlichkeit mit der Zusammensetzung der aktuellen Flüssigkeitsprobe aufweist. Somit wird die höchste Messgüte erreicht, wenn zur Bestimmung des aktuellen CSB-Messwerts die Kalibrierparameter bzw. die Kalibrierfunktion herangezogen werden die dieser Referenzprobe hinterlegt sind. Mittels dieser neuen Kalibrierparameter bzw. der neuen Kalibrierfunktion kann aus dem aktuell gemessenen Absorptionsspektrum ein korrigierter Messwert bestimmt werden

Weitere Kalibrierparameter, Prüfparameter und zugehörige Kriterienfunktionen für weitere Referenzproben können im Laufe der Zeit in den Speicher der spektrometrischen Vorrichtung geladen werden. Auf diese Weise kann die Verlässlichkeit der Messwerte immer weiter verbessert werden.

## Patentansprüche

1. Verfahren zur Bestimmung eines Parameters, insbesondere des chemischen Sauerstoffbedarfs (CSB) oder des organischen Gesamtkohlenstoffgehalts (TOC), einer Flüssigkeitsprobe mittels einer spektrometrischen Vorrichtung, umfassend die Schritte:
Ermitteln eines Absorptionsspektrums der Flüssigkeitsprobe mindestens in einem vorgegebenen Wellenlängenbereich;
Ermitteln eines Messwerts des Parameters der Flüssigkeitsprobe aus dem Absorptionsspektrum im vorgegebenen Wellenlängenbereich;
Vergleichen des ermittelten Absorptionsspektrums im vorgegebenen Wellenlängenbereich mit mindestens einem Referenz-Absorptionsspektrum mindestens einer Referenzprobe bekannter Zusammensetzung;
Anhand des Vergleichs Bestimmen mindestens eines Ähnlichkeitsparameters, der die Ähnlichkeit zwischen dem ermittelten Absorptionsspektrum und dem Referenz-Absorptionsspektrum repräsentiert;
Klassifizieren des ermittelten Messwerts anhand des mindestens einen Ähnlichkeitsparameters.

2. Verfahren nach Anspruch 1,
wobei der Schritt des Vergleichens des ermittelten Absorptionsspektrums im vorgegebenen Wellenlängenbereich mit mindestens einem Referenz-Absorptionsspektrum einer Referenzprobe bekannter Zusammensetzung folgende Schritte umfasst:
Ermitteln von Ist-Werten mindestens eines Satzes von vorgegebenen Prüfparametern aus dem ermittelten Absorptionsspektrum;
Vergleichen des ermittelten Satzes von Ist-Werten mit mindestens einem hinterlegten Satz,
insbesondere mit mehreren hinterlegten Sätzen, von Soll-Werten der Prüfparameter für den ermittelten Messwert des Parameters der Flüssigkeitsprobe oder eines damit korrelierten Werts,
wobei die Prüfparameter des Satzes von Prüfparametern aus dem Referenz- Absorptionsspektrum der Referenzprobe abgeleitet sind, und wobei die Prüfparameter derart vorgegeben sind, dass sie charakteristische Eigenschaften des Referenz-Absorptionsspektrums repräsentieren.

3. Verfahren nach Anspruch 2,
wobei der Schritt des Bestimmens mindestens eines Ähnlichkeitsparameters anhand des Vergleichs folgende Schritte umfasst:
Bestimmen von Ähnlichkeitsparametern aus einem Vergleich der Ist-Werte für die Prüfparameter mit den entsprechenden hinterlegten Soll-Werten der Prüfparameter.

4. Verfahren nach einem der Ansprüche 2 oder 3,
wobei als Prüfparameter ein oder mehrere der für das Referenz-Absorptionsspektrum charakteristischen Parameter verwendet werden:
eine Wellenlänge eines Maximums, eine Wellenlänge eines Minimums, einen Absorptionswert oder
einen Intensitätswert eines Maximums, einen Absorptionswert oder einen Intensitätswert eines Minimums, einen Wellenlängenbereich, in dem die Absorption einer Basislinie des Absorptionsspektrums entspricht, eine erste Ableitung in einem vorgegebenen Punkt des Absorptionsspektrums, eine Differenz oder ein Verhältnis von Absorptions- oder Intensitätswerten bei ausgewählten Wellenlängen des Referenz-Absorptionsspektrums, insbesondere von Absorptionswerten oder Intensitätswerten von absoluten oder lokalen Minima oder Maxima oder von Intervallgrenzen bestimmter Wellenlängenbereiche

5. Verfahren nach einem der Ansprüche 2 bis 4,
wobei unterschiedliche hinterlegte Sätze von Prüfparametern charakteristische Merkmale von Referenz-Absorptionsspektren unterschiedlicher Referenzproben repräsentieren.

6. Verfahren nach einem der Ansprüche 2 bis 5,
wobei der Soll-Wert eines Prüfparameters in Form einer Kriterienfunktion hinterlegt ist, wobei die Kriterienfunktion den Prüfparameter als Funktion des Parameters der Flüssigkeitsprobe oder einer daraus abgeleiteten Konzentration angibt, und wobei als Soll-Wert der Funktionswert der Kriterienfunktion für den als Messwert ermittelten Wert des Parameters der Flüssigkeitsprobe oder der daraus abgeleiteten Konzentration verwendet wird.

7. Verfahren nach einem der Ansprüche 2 bis 6,
wobei zu den Soll-Werten zusätzlich ein Toleranzintervall hinterlegt ist, und wobei der Ähnlichkeitsparameter als prozentuale Abweichung des Ist-Werts vom Soll-Wert bezogen auf das Toleranzintervall bestimmt wird.

8. Verfahren nach einem der Ansprüche 1 bis 7,
wobei aus den ermittelten Ähnlichkeitsparametern Gütewerte abgeleitet werden, aus denen auf die Messgüte des Messwerts geschlossen wird, wobei insbesondere aus den Gütewerten ein Bewertungsparameter ermittelt wird.

9. Verfahren nach Anspruch 8,
wobei Wertebereiche für die Gütewerte vorgegeben sind, anhand derer der Messwert hinsichtlich seiner Messgüte klassifiziert wird, wobei insbesondere Schwellenwerte und/oder Wertebereiche für den Bewertungsparameter vorgegeben sind, anhand derer der Messwert hinsichtlich seiner Messgüte klassifiziert wird.

10. Verfahren nach Anspruch 8 oder 9,
wobei die Gütewerte und/oder der Bewertungsparameter zusammen mit dem Messwert des Parameters ausgegeben werden.

11. Verfahren nach einem der Ansprüche 1 bis 10,
wobei das Vergleichen des ermittelten Absorptionsspektrums im vorgegebenen Wellenlängenbereich mit mindestens einem Referenz-Absorptionsspektrum einer Referenzsubstanz, insbesondere zur Durchführung eines Tests des Verfahrens,
oder mit einem Referenz-Absorptionsspektrum einer Referenzprobe bekannter Zusammensetzung, beispielsweise einer Abwasserprobe,
oder mit Referenz-Absorptionsspektren mehrerer Referenzproben unterschiedlicher Zusammensetzung, beispielsweise unterschiedlich zusammengesetzter Abwasserproben, durchgeführt wird.

12. Verfahren nach einem der Ansprüche 1 bis 11,
wobei ein Vergleich des Absorptionsspektrums der Flüssigkeitsprobe im vorgegebenen Wellenlängenbereich mit Referenz-Absorptionsspektren mehrerer Referenzproben unterschiedlicher Zusammensetzung durchgeführt wird, und wobei dasjenige Referenz-Absorptionsspektrum ermittelt wird, das dem Absorptionsspektrum der Flüssigkeitsprobe am ähnlichsten ist.

13. Verfahren nach Anspruch 12,
wobei eine Angabe zu der Referenzprobe, deren Referenz-Absorptionsspektrum im vorgegebenen Wellenlängenbereich die größte Ähnlichkeit mit dem Absorptionsspektrum der Flüssigkeitsprobe aufweist, ausgegeben wird.

14. Verfahren nach Anspruch 12,
wobei zu jeder Referenzprobe aus Kalibriermessungen mit der Referenzprobe gewonnene Kalibrierparameter hinterlegt sind, und wobei ein korrigierter Messwert des Parameters der Flüssigkeitsprobe unter Verwendung der zu der Referenzprobe hinterlegten Kalibrierparameter bestimmt wird, deren Referenz-Absorptionsspektrum dem ermittelten Absorptionsspektrum der Flüssigkeitsprobe am ähnlichsten ist.

15. Verfahren nach Anspruch 12,
wobei zu dem ermittelten Referenz-Absorptionsspektrum hinterlegte Kriterienfunktionen, welche Verläufe von vorgegebenen Prüfparametern als Funktion des Parameters der Flüssigkeitsprobe oder einer daraus abgeleiteten Konzentration angeben, variiert werden,
bis ein oder mehrere Ähnlichkeitsparameter, die aus einem Vergleich von einem oder mehreren Soll-Werten der vorgegebenen Prüfparameter, mit einem oder mehreren Ist-Werten der vorgegebenen Prüfparameter ermittelt werden, maximal sind,
wobei die Soll-Werte für den aus dem Absorptionsspektrum der Flüssigkeitsprobe ermittelten Messwert des Parameters der Flüssigkeitsprobe oder für einen damit korrelierten Wert aus den Kriterienfunktionen abgeleitet werden, und
wobei die Ist-Werte aus dem Absorptionsspektrum der Flüssigkeitsprobe bestimmt werden.
